# EUROPEAN PATENT SPECIFICATION

(11) **EP 1 796 556 B1**
(45) Date of publication and mention of the grant of the patent: **26.06.2013**
(21) Application number: 05776169.4
(22) Date of filing: 10.08.2005
(51) Int. Cl.: A61B 17/132

(54) **IMPROVEMENTS IN TOURNIQUETS**
VERBESSERUNGEN VON STAUMANSCHETTEN
AMELIORATIONS APPORTEES AUX TOURNIQUETS

(30) Priority: 12.08.2004 GB 0417949
(43) Date of publication of application: 20.06.2007
(73) Proprietor: Imperial Innovations Limited, Imperial College London SW7 2BT (GB)
(72) Inventor: KERSTEIN, Ryan, London SW6 1TN (GB); FELLOWES, Christian, London W6 9ET (GB)
(74) Representative: Hocking, Adrian Niall
(86) International application number: PCT/EP2005/053950
(87) International publication number: WO 2006/015987

(56) References cited:
- US-A- 3 930 506
- US-A- 4 215 687
- US-A- 5 540 714
- US-A1- 2004 097 902

## Description

### Field of the invention

This invention relates to a disposable tourniquet.

### Background of the invention

The use of tourniquets in a clinical environment is primarily for the purpose of limiting venous blood flow in a limb to ensure vessel distension, to assist in venepuncture and cannulation procedures, also referred to as blood work. There are currently two types of tourniquets in common use, namely reusable and disposable. Re-usable tourniquets, the most common having the form of a length of elasticated fabric, provide excellent performance in terms of their ease of use and patient comfort. However, studies have shown that ⁺he re-use of a tourniquet poses a risk of infections being passed from one patient to another.

In order to overcome this problem, various disposable tourniquets have been developed. However, while reducing the risk of cross-infection, such tourniquets have not performed as well as re-usable tourniquets and they can be uncomfortable for patients.

U.S. Pat No. 5219356 describes a disposable tourniquet comprising an elongated, flat, stretchable band. The band has a pressure sensitive adhesive face on one side of the band at one end, and a release agent on the same face of the band but spaced from the adhesive face. The tourniquet is stored with the adhesive face folded against and adhering to the release agent and is deployed by peeling the adhesive face away from the release agent, wrapping the tourniquet about the arm and pulling it sufficiently tight and then adhering the adhesive face of the outside surface of the band to hold the tourniquet in place.

It is a disadvantage of this known tourniquet that it is difficult to set the correct tension before the adhesive face is stuck down. The user has to stretch the tourniquet to the extent necessary to achieve the correct tension before adhering the free end of the tourniquet to the band surrounding the patient's arm. While performing this task, the user is also hampered by only being able to grip one end of the band, the other end being trapped beneath the loop encircling the patient's arm. If the tension should prove to be insufficient then the veins of the patient will not be sufficiently distended and if the tension is excessive then the patient can be caused unnecessary discomfort.

US2004/0097902 describes a device intended to apply gentle compression to a phallus to facilitate intracavemosal anaesthesia. The band is elasticated and its ends can be secured to one another by means of a hook and loop closure after the desired tension has been applied.

US 4,215,687 describes a support bandage made up a set of strips that are held parallel to one another to form a wide strap. Each strip is elasticated and its ends can be secured to one another, once again by a hook and loop fastener, after the desired tension has been set.

US 5,540,714 describes a disposable tourniquet formed from a strip of elastic fabric material. Such fabric may be initially selected or treated with a polymeric or elastomeric material to provide surfaces having predetermined frictional characteristics to minimize slippage and discomfort to the patient and to facilitate ease of use in knotting and releasing the tourniquet.

### Object of the invention

The present invention seeks to provide a tourniquet which is sufficiently inexpensive to be disposable and which makes it easier to set the correct tension while minimising patient discomfort.

### Summary of the invention

According to claim 1 of the present invention, there is provided a disposable tourniquet comprising a band of substantially inextensible material having an aperture, a grip region disposed between the aperture and one end of the band, a fastening region sufficiently narrow to pass through the aperture, a loop region having sufficient length to encircle a limb and disposed between the aperture and the fastening region, characterised by an adhesive for adhering overlapping regions of the band to one another after the loop region has been wrapped around a limb and the fastening region has been passed through the aperture, the substantially inextensible material is plasticised paper material.

Mailing envelopes are currently available, for example under the trade mark Tyvek^{®}, which are made of a plasticised paper that does not tear and is inextensible. The invention is predicated on the realisation that such a material is well suited to use as a tourniquet.

According to claim 8 of the present invention there is provided a tourniquet dispenser comprising a sterile box and a strip of inextensible plasticised paper material defining a plurality of disposable tourniquets detachably connected to one another end to end, wherein each tourniquet in the strip comprises a band having an aperture, a grip region disposed between the aperture and one end of the band, a fastening region sufficiently narrow to pass through the aperture, a loop region having sufficient length to encircle a limb and disposed between the aperture and the fastening region, and a pressure sensitive adhesive for adhering overlapping regions of the band to one another after the loop region has been wrapped around a limb and the fastening region has been passed through the aperture, and wherein the strip is wound into a roll and packaged in a the said sterile box from which the elongate continuous tourniquet strip can be drawn out, to reveal a disposable tourniquet one at a time, and separation from the remainder of the roll.

In this aspect of the invention, each tourniquet is formed as part of an elongate continuous strip within which individual tourniquets are detachably connected to one another end to end. Such a continuous strip is formed into a roll and packaged in the dispenser from which tourniquets can be drawn out, one at a time. In this way, it is possible to ensure that each tourniquet remains sterile until the time that it is dispensed.

The length of the grip region between the aperture and the end of the band should be sufficient to ensure that it can be gripped firmly while the band is being tensioned around the limb of a patient. The dimensions of the aperture must be sufficient to allow the tensioning region to pass through it while leaving sufficient material around the aperture to ensure that the band does not tear while it is being tensioned.

The adhesive used to secure the tourniquet after it has been wrapped round a limb need not be particularly strong as it only has to withstand shearing forces, there being no tendency for the ends of the tourniquet to peel apart. Consequently, it is possible to use a pressure sensitive adhesive as used, for example, in mailing envelopes. An advantage of this is that after any blood work has been terminated, it is possible to remove the tourniquet easily by using a peeling action to separate the regions adhering to one another.

A protective liner coated with a release agent may be used to cover the adhesive during storage but such a cover may not be required if the tourniquets are wound into a continuous supply roll. The dimensions of a liner, if present, should be at least equal to those of the adhesive but it is preferred to provide a pull tab to ease peeling of the liner away from the adhesive during deployment of the tourniquet.

### Brief description of the drawings

The invention will now be described further, by way of example only, with reference to the accompanying drawings, in which:
Figure 1 is a plan view of a tourniquet of the present invention, and
Figure 2 shows the tourniquet of Figure 1 in the position that it would adopt when wrapped around a limb of a patient.

### Detailed description of the preferred embodiment

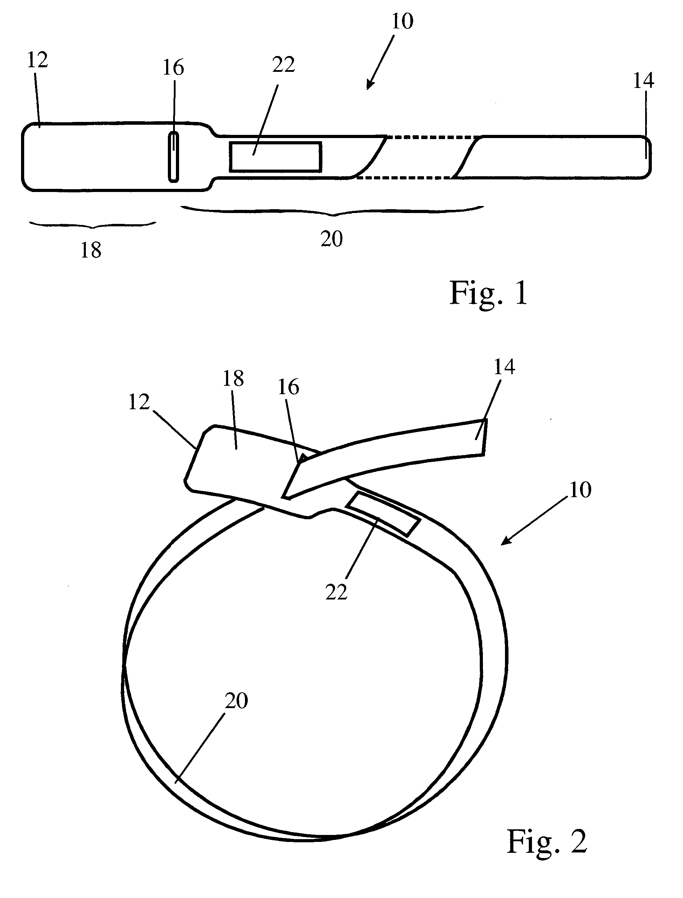

The tourniquet 10 in figure 1 is formed as a band of material that is substantially inextensible. The material is preferably a plasticised paper as used in Tyvek® mailing envelopes.

The band has a wider end 12 and a narrower end 14. An aperture 16 is spaced from the wider end 12 by means of a region 18 that acts as a gripping tab and will herein be termed the grip region. To the right of the aperture 16, as viewed, there is provided a region 22 coated with an adhesive. The narrow end 14 of the band 10 is of a sufficient width to pass through the aperture 16 and will herein be termed the fastening region. The region of the band 10 designated 20 in Figure 1, which extends between the fastening region 14 and the aperture 16 constitutes the loop that is wrapped around the limb of the patient and is herein termed the loop region.

The tourniquet of Figure 1 is deployed in a manner most readily understood by reference to figure 2. The loop region 20 of the band is wrapped around the limb of the patient, usually the upper arm. The fastening region 14 is next threaded through the aperture of 16, as shown in the drawing. By pulling on the grip region 12 and the fastening region 14, it is possible to tension the loop region 20 of the tourniquet to the point where the required distension of the veins occurs. The fastening region 14 may now be brought down and adhered to adhesive region 22 to hold the tourniquet in place.

It is an important advantage of the illustrated tourniquet that it minimises discomfort to the patient. In particular, one can ensure that the correct level of tension has been applied before the ends of the tourniquet are adhered to one another. In this way, it is possible to set the level of tension to the minimum necessary to achieve the required distension of the veins. Furthermore, all parts of the tourniquet used to apply tension around the limb lie in the same plane as one another and there is therefore no tendency to pinch the skin of the patient as the tourniquet is tightened.

The adhesive region 22 need not necessarily be located in the position illustrated. For example, it would be alternatively possible for an adhesive region to be formed on the underside of the grip portion 12 or the fastening portion 14. The adhesive may be permanently exposed or it may be covered with a liner until such time as the tourniquet is to be deployed. Once again this is somewhat analogous to the adhesive found in mailing envelopes.

It is preferred to form the tourniquet 10 as part of a continuous strip wound into a roll and stored within a sterile box, from which only one end of roll protrudes. In this way, it is possible to ensure that the tourniquet remains sterile until such time as it is to be deployed. If tourniquets are packaged as a continuous strip, it is not necessarily to protect the adhesive region 22 with a liner as the turns of the continuous strip will ensure that the adhesive remains covered while it is in storage. Once a tourniquet has been torn off the strip, the adhesive region 22 can be used to stick its ends to each other to hold the tourniquet in place while blood work is being carried out.

After termination of the blood work, the tourniquet can be removed by simply peeling away the fastening region 14 from the adhesive region 22 and pulling on the grip region 12 to release the tourniquet from the patient's limb. The tourniquet can then be discarded to avoid any risk of contaminating another patient.

## Claims

1. A disposable tourniquet comprising a band (10) of substantially inextensible material having
an aperture (16),
a grip region (18) disposed between the aperture (16) and one end (12) of the band,
a fastening region (14) sufficiently narrow to pass through the aperture (16),
a loop region having sufficient length to encircle a limb and disposed between the aperture (16) and the fastening region (14), **characterised by**
an adhesive (22) for adhering overlapping regions of the band to one another after the loop region (20) has been wrapped around a limb and the fastening region (14) has been passed through the aperture (16), and
the substantially inextensible material is plasticised paper material.

2. A tourniquet as claimed in Claim 1, wherein the adhesive is a pressure sensitive adhesive.

3. A tourniquet as claimed in Claim 2, wherein the adhesive is covered by means of a protective liner coated with a release agent.

4. A tourniquet as claimed in Claim 3, wherein the liner includes a pull tab to ease peeling of the liner away from the adhesive during deployment of the tourniquet.

5. A tourniquet as claimed in any preceding claim, wherein the aperture extends transversely across the band.

6. A tourniquet as claimed in any one of claims 1 to 4, wherein the aperture extends parallel to the length of the band.

7. An elongate continuous tourniquet strip comprising a plurality of disposable tourniquets as claimed in any preceding claim, the said disposable tourniquets being detachably connected to one another end to end.

8. A tourniquet dispenser comprising a sterile box and a strip of inextensible plasticised paper material defining a plurality of disposable tourniquets detachably connected to one another end to end, wherein each tourniquet in the strip comprises a band (10) having an aperture (16), a grip region (18) disposed between the aperture (16) and one end of the band (10), a fastening region (14) sufficiently narrow to pass through the aperture (16), a loop region having sufficient length to encircle a limb and disposed between the aperture (16) and the fastening region (14), and a pressure sensitive adhesive (22) for adhering overlapping regions of the band (10) to one another after the loop region has been wrapped around a limb and the fastening region (14) has been passed through the aperture (16), and wherein the strip is wound into a roll and packaged in the said sterile box from which the elongate continuous tourniquet strip can be drawn out, to reveal a disposable tourniquet one at a time for separation from the remainder of the roll.

## Patentansprüche

1. Einweg-Aderpresse, umfassend ein Band (10) aus im Wesentlichen nicht entfaltbarem Material, umfassend
eine Öffnung (16),
einen Griffbereich (18), der zwischen der Öffnung (16) und einem Ende (12) des Bands angeordnet ist,
einen Befestigungsbereich (14), der ausreichend eng ist, um durch die Öffnung (16) zu passieren,
einen Schleifenbereich, der ausreichend lang ist, um eine Gliedmaße zu umgeben, und zwischen der Öffnung (16) und dem Befestigungsbereich (14) angeordnet ist, **gekennzeichnet durch**
ein Haftmittel (22) zum Haften von überlappenden Bereichen des Bands aufeinander, nachdem der Schleifenbereich (20) um eine Gliedmaße gewickelt wurde und der Befestigungsbereich (14) **durch** die Öffnung (16) passiert wurde,
und das im Wesentlichen nicht entfaltbare Material plastifiziertes Papiermaterial ist.

2. Aderpresse nach Anspruch 1, wobei das Haftmittel ein druckempfindliches Haftmittel ist.

3. Aderpresse nach Anspruch 2, wobei das Haftmittel mit Hilfe einer Schutzverkleidung, beschichtet mit einem Freisetzungsmittel, bedeckt ist.

4. Aderpresse nach Anspruch 3, wobei die Verkleidung eine Zuglasche umfasst, um das Abziehen der Verkleidung vom Haftmittel während der Entfaltung der Aderpresse zu erleichtern.

5. Aderpresse nach einem der vorhergehenden Ansprüche, wobei sich die Öffnung quer über das Band erstreckt.

6. Aderpresse nach einem der Ansprüche 1 bis 4, wobei sich die Öffnung parallel zur Länge des Bands erstreckt.

7. Verlängerter kontinuierlicher Aderpressstreifen, umfassend eine Vielzahl von Einweg-Aderpressen nach einem der vorhergehenden Ansprüche, wobei die Einweg-Aderpressen abnehmbar miteinander Ende an Ende verbunden sind.

8. Aderpressen-Spender, umfassend eine sterile Box und einen Streifen aus nicht entfaltbarem Papiermaterial, umfassend die Definition einer Vielzahl von Einweg-Aderpressen, die abnehmbar miteinander Ende an Ende verbunden sind, wobei jede Aderpresse im Streifen ein Band (10) mit einer Öffnung (16) umfasst, einen Griffbereich (18), der zwischen der Öffnung (16) und einem Ende des Bands (10) angeordnet ist, einen Befestigungsbereich (14), der ausreichend eng ist, um durch die Öffnung (16) zu passieren, einen Schleifenbereich, der ausreichend lang ist, um eine Gliedmaße zu umgeben, und zwischen der Öffnung (16) und dem Befestigungsbereich (14) angeordnet ist, und ein druckempfindliches Haftmittel (22), um überlappende Bereiche des Bands (10) aneinander zu haften, nachdem der Schleifenbereich um eine Gliedmaße gewickelt wurde, und der Befestigungsbereich (14) durch die Öffnung (16) passiert wurde, und wobei der Streifen in einer Rolle gewickelt und in die sterile Box verpackt wird, aus der der verlängerte kontinuierliche Aderpressstreifen herausgezogen werden kann, um eine Einweg-Aderpresse nach der anderen aufzudecken, um sie von der restlichen Rolle zu trennen.

## Revendications

1. Tourniquet jetable comprenant une bande (10) de matériau sensiblement inextensible ayant
une ouverture (16),
une région de préhension (18) disposée entre l'ouverture (16) et un bout (12) de la bande,
une région de fixation (14) suffisamment étroite pour passer à travers l'ouverture (16),
une région de boucle ayant une longueur suffisante pour encercler un membre et disposée entre l'ouverture (16) et la région de fixation (14), **caractérisé par**
un adhésif (22) pour faire adhérer des régions chevauchantes de la bande l'une à l'autre après que la région de boucle (20) a été enroulée autour d'un membre et que la région de fixation (14) a été passée à travers l'ouverture (16), et
le matériau sensiblement inextensible est un matériau en papier plastifié.

2. Tourniquet selon la revendication 1, dans lequel l'adhésif est un adhésif sensible à la pression.

3. Tourniquet selon la revendication 2, dans lequel l'adhésif est recouvert au moyen d'un revêtement protecteur revêtu d'un agent antiadhérent.

4. Tourniquet selon la revendication 3, dans lequel le revêtement comprend une languette de préhension pour faciliter le pelage du revêtement vis-à-vis de l'adhésif durant la mise en place du tourniquet.

5. Tourniquet selon l'une quelconque des revendications précédentes, dans lequel l'ouverture s'étend transversalement en travers de la bande.

6. Tourniquet selon l'une quelconque des revendications 1 à 4, dans lequel l'ouverture s'étend parallèle à la longueur de la bande.

7. Bande de tourniquets continue allongée comprenant une pluralité de tourniquets jetables selon l'une quelconque des revendications précédentes, lesdits tourniquets jetables étant reliés de manière détachable les uns aux autres bout à bout.

8. Distributeur à tourniquets comprenant une boîte stérile et une bande de matériau en papier plastifié inextensible définissant une pluralité de tourniquets jetables reliés de manière détachable les uns aux autres bout à bout, chaque tourniquet dans la bande comprenant une bande (10) ayant une ouverture (16), une région de préhension (18) disposée entre l'ouverture (16) et un bout de la bande (10), une région de fixation (14) suffisamment étroite pour passer à travers l'ouverture (16), une région de boucle ayant une longueur suffisante pour encercler un membre et disposée entre l'ouverture (16) et la région de fixation (14), et un adhésif sensible à la pression (22) pour faire adhérer des régions se chevauchant de la bande (10) l'une à l'autre après que la région de boucle a été enroulée autour d'un membre et que la région de fixation (14) a été passée à travers l'ouverture (16), et la bande étant enroulée en un rouleau et conditionnée dans ladite boîte stérile de laquelle la bande de tourniquets continue allongée peut être tirée, pour révéler un tourniquet jetable un à la fois pour séparation du reste du rouleau.
